# EUROPEAN PATENT SPECIFICATION

(11) **EP 2 323 721 B1**
(45) Date of publication and mention of the grant of the patent: **22.06.2016**
(21) Application number: 09771905.8
(22) Date of filing: 02.07.2009
(51) Int. Cl.: A61M 16/00, A61F 5/56, A61B 5/08, A61B 5/103, A61B 5/0205, A61B 7/00

(54) **EXTENDABLE AIR DELIVERY SYSTEM**
ERWEITERBARES LUFTZUFUHRSYSTEM
SYSTÈME DE DISTRIBUTION D'AIR EXTENSIBLE

(30) Priority: 03.07.2008 CN 200810135704
(43) Date of publication of application: 25.05.2011
(73) Proprietor: Chou, Chang-An, Taiwan (TW)
(72) Inventor: Chou, Chang-An, Taiwan (TW)
(74) Representative: 2K Patentanwälte Blasberg Kewitz & Reichel
(86) International application number: PCT/CN2009/000749
(87) International publication number: WO 2010/000135

(56) References cited:
- CN-A- 101 106 941
- US-A- 5 735 267
- US-A1- 2005 038 353
- US-A1- 2006 070 625
- US-A1- 2007 000 494
- US-A1- 2007 193 583
- US-A1- 2007 208 269
- US-B1- 6 305 372

## Description

### FIELD OF THE INVENTION

The present invention is related to an extendable air delivery system and an air delivery method, and more particularly to an air delivery system which can receive extendable/replaceable physiological signal sources for adjusting the delivery pressure so as to meet patients' different demands.

### BACKGROUND OF THE INVENTION

Obstructive sleep apnea syndrome (OSAS) is a cessation of oronasal airflow caused by narrowed or collapsed upper airway.

The most commonly prescribed treatment for obstructive sleep apnea is to provide a continuous positive airway pressure during the sleep. A positive airway pressure device (PAP device) delivers air pressure through a nasal/oralnasal mask that the patient wears while sleeping. The pressure keeps the throat open for eliminating obstructive apneas and allowing the patient to breathe normally for whole night and to keep sleep uninterruptedly and restoratively.

One common type of PAP devices is CPAP device which provides a constant air delivery pressure. Other types of PAP devices are also available. For example, one is BiPAP (Bilevel PAP) device which provides two positive pressures, a lower pressure for the patient's expiration and a higher pressure for the inspiration. Another one is APAP (Auto PAP) device which automatically detects the patient's apnea/hypopnea and alters the air pressure according thereto. A further one is VPAP (Variable PAP) device which adjusts the air pressure in accordance with the patient's breathing pattern automatically.

US 2007/208269 A1 discloses methods, systems and mask assemblies for use in determining the occurrence of respiratory events using a frontal electrode array. The methods, systems and mask assemblies involve use of means for flow measurement of breathing gas of a person, blood oxygen saturation measurement means and a frontal electrode array for measuring frontal bioelectric signals, each of which is coupled to a processing unit. The processing unit is configured to determine the occurrence of at least one of an apnea event and a hypopnea event based on the measurement signals. Some embodiments involve calculation of an apnea-hypopnea index (AHI) based on the determined apnea and hypopnea events over a period of time.

All these developments are focused on adjusting the air pressure based on the patient's breathe, and thus, preventing improper air delivery pressure. Insufficient delivery pressure might be unable to open the airway, and excess delivery pressure might bring the patient uncomfortability, and also, cause mask leakage and/or arousal which on the contrary will influence the patient's sleep. Therefore, how to find out the minimum effective delivery pressure for the patient's most comfortability is one of the main goals in PAP development, and an example can be seen in US 6,349,724.

However, as known, most PAP devices have a fixed hardware structure which limits the adaptation to the patient's different demands. Some improvements for this limitation are US 2007/0193583, which increases preloaded programs to provide multiple delivery modes, and US 2007/0023045, which provides the possibility to alter the hardware, and further, US 6,397,845 and US 7,204,250 provide the information about sleep stage according to the physiological signals for being the basis of air pressure adjustment.

The object of the present invention is to provide an extendable air delivery system by providing a PAP device and at least an external sensing device connected to the PAP device, so as to increase flexibility in hardware arrangement.

Another object of the present invention is to provide an extendable air delivery system, wherein the external sensing device provides a signal/data based on the physiological signals acquired thereby to the PAP device for adjusting the air delivery pressure, so that through adopting the appropriate sensing device according to the patient's requirement, the air delivery pressure can have a corresponding variation.

Another further object of the present invention is to provide an extendable air delivery system in which the PAP device can perform a preset air delivery behavior even as not connecting with the external sensing device.

Still another object of the present invention is to provide an extendable air delivery system which can regulate the air delivery pressure to be as close as the minimum effective delivery pressure, so as to accordingly provide the comfortability of usage, and at the same time, reduce the occurrences of arousal and other side effects.

### SUMMARY OF THE INVENTION

As described above, the improvements of PAP device are mainly focused on optimizing the delivery pressure to maximize the comfortability and reduce the influences caused by improper delivery pressure.

In one aspect of the present invention, it provides an extendable air delivery system, including a PAP device and a sensing device. The PAP device includes a flow generator for providing a breathable air to a patient, a processor for providing a control signal to the flow generator, and a digital communication module controlled by the processor. The sensing device includes at least a sensor for acquiring a physiological information from the patient, a processor for obtaining the physiological information from the sensor and generating a signal/data according thereto, and a digital communication module controlled by the processor. And, the PAP device is configured to be one selected from a CPAP device, a BiPAP device, an APAP device and a VPAP device, and the PAP device and the sensing device are configured to be connected to or separated from each other; and wherein
when the sensing device is not connected with the PAP device, the PAP device enters a preset independent operation mode and performs a preloaded first air delivery behavior of CPAP device, or BiPAP device, or APAP device, or VPAP device;
when the sensing device is connected with the PAP device, the PAP device enters a common operation mode, and during the common operation mode, the PAP device and the sensing device communicate via the corresponding digital communication modules thereof, for sending the signal/data from the sensing device to the PAP device, thereby deciding a second air delivery behavior of the flow generator, wherein the second air delivery behavior is an air delivery behavior of BiPAP device, or APAP device, or VPAP device; and
the PAP device is configured to check the connection with the sensing device to decide the operation mode.

Here, the digital communication modules of the PAP device and the sensing device can be implemented to perform wired and/or wireless communication with each other or with an external device, such as, a computer or a handheld device.

Preferably, the air delivery of the flow generator is adjusted depending on the information provided by the sensing device for obtaining the optimized delivery pressure, so as to provide an using experience as comfortable as possible.

Here, the physiological information includes, but not limited, respiratory cycle, the occurrence of sleep apnea/hypopnea, sleep stages and body position. And, the sensor can be, but not limited, airflow sensor, respiratory effort belts, flow/pressure sensor assembled with the mask, flow/pressure sensor assembled with the tubing, snore sensor, oximeter, heart rate detector, body position sensor, limb movement sensor, ECG electrodes, EEG electrodes, EOG electrodes, and/or EMG electrodes.

And further, the numbers and the types of both the sensing device and the sensor are also not restricted. It can be plural sensing devices to cooperate with the PAP device, and/or plural sensors connected to the sensing device, which is depending on the patient's requirement.

Then, in a preferred embodiment, the PAP device/the sensing device is implemented to show the therapeutic result after each treatment, for example, RDI (Respiratory Disturbance Index)/ AHI(Apnea/Hypopnea Index), so that the patient can clearly understand if the treatment is effective and if the selected sensing device (and/or the sensor) is appropriate. And, according to the result, the patient can replace the sensing device (and/or the sensor) or add other sensing device(s) (and/or sensor(s)) for adjusting the air delivery behavior so as to improve the treatment.

Moreover, the PAP device/the sensing device can further include a memory for storing, for example, but not limited, the settings, the information about respiration and air pressure during the treatment, RDI/ AHI, and other related information, which benefits the follow-up evaluation.

In the present invention, in addition to the sensing device provides the signal/data to the PAP device, oppositely, the PAP device also can provide the air delivery related information to the sensing device for involving in the generation of the signal/data.

Furthermore, the air delivery system of the present invention also can connect to the network for further extension. For example, through the network download, the PAP device or the sensing device both can alter the settings/operation thereof to match to different patients or physiological conditions, and through the network upload, the settings, the operation records and the related data all can be transmitted to the remote medical personnel, so that the consultation about the treatment and the modification of the settings can be performed in an easier and more effective way.

As known, the PAP device is quite expensive and the patient might not afford to buy another PAP device as the demand changes. Through the architecture of the present invention, the demand variation can be matched by changing the sensing device, so as to save the cost. Further, since the contents of the signal/data are based on the type and the number of the sensor(s), it will be even more economic to change the sensor(s) only.

Furthermore, the extendable architecture of the present invention is particularly beneficial to the titration process of the PAP device. Through varying the type and the number of the sensing device, the system can be adapted to the patient's different physiological conditions for optimizing the air delivery behavior. And, the titration process can even be more simplified through a real time modification from the external device communicated therewith. Plus, further through the network connection capability, the patient can execute the titration process at home and transmit the related data to the doctor/technician or the website (with analysis algorithm) for evaluation, or even the titration process can be monitored by a remote medical personnel in real time.

The present invention is also related to a physiological signal acquisition device including at least a sensor for acquiring a physiological information from the patient, and a processor for obtaining and analyzing the physiological information from the sensor to produce an analysis result. And, particularly, the physiological signal acquisition device further includes a digital communication module for communicating with a PAP, which supplies a breathable pressured air to a patient, so that a signal/data generated according to the received physiological information can be transmitted to the PAP device, thereby controlling the pressure of the breathable air.

Besides, based on the independency of the PAP device and the sensing device, the sensing device can be used alone while the PAP device is idle, so as to provide the function of home monitoring, and through further cooperating with an external processing device, a computer (with a network), an analysis result can be obtained. Therefore, the present invention provides a multifunctional architecture.

### BRIEF DESCRIPTION OF THE DRAWINGS

A more detailed understanding of the invention may be had from the following description of a preferred embodiment, given by way of example, and to be understood in conjunction with the accompanying drawings, wherein:
Fig. 1 is a circuit block showing the PAP device and the sensing device according to the present invention
Fig. 2A is a schematic view showing the PAP device in a preferred embodiment of the present invention;
Fig. 2B is a schematic view showing the sensing device in a preferred embodiment of the present invention;
Fig. 3 is a is a flow chart showing the operation of the extendable air delivery system of the present invention;
Fig. 4 is a schematic view showing an exemplary application of the present invention; and
Fig. 5 is a schematic view showing another exemplary application of the present invention.

### DETAILED DESCRIPTION OF THE PREFERRED EMBODIMENTS

According to the concept of the present invention, the extendable air delivery system includes two portions: a PAP device and at least an external sensing device. The PAP device is used to provide a breathable pressured air to the patient which can operate independently or cooperate with the external sensing device. The sensing device is communicated with the PAP device for determining the operation of the air delivery system together.

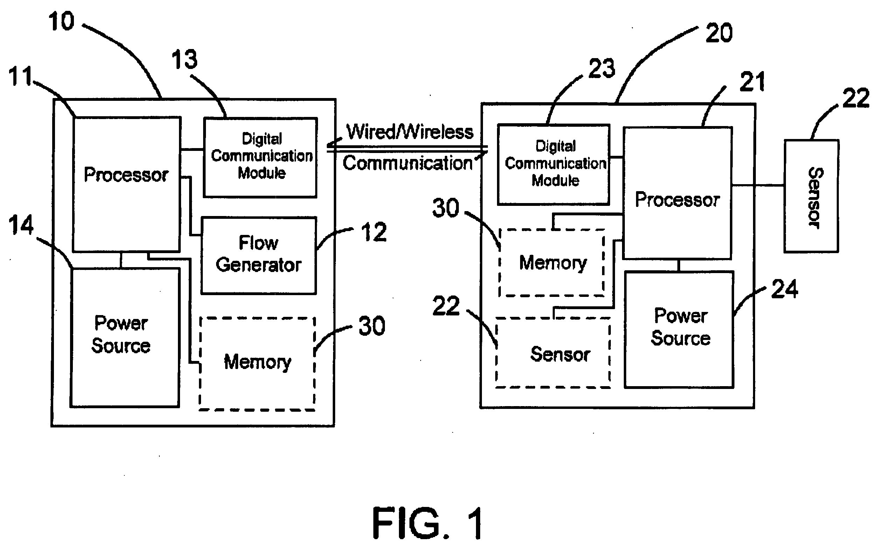

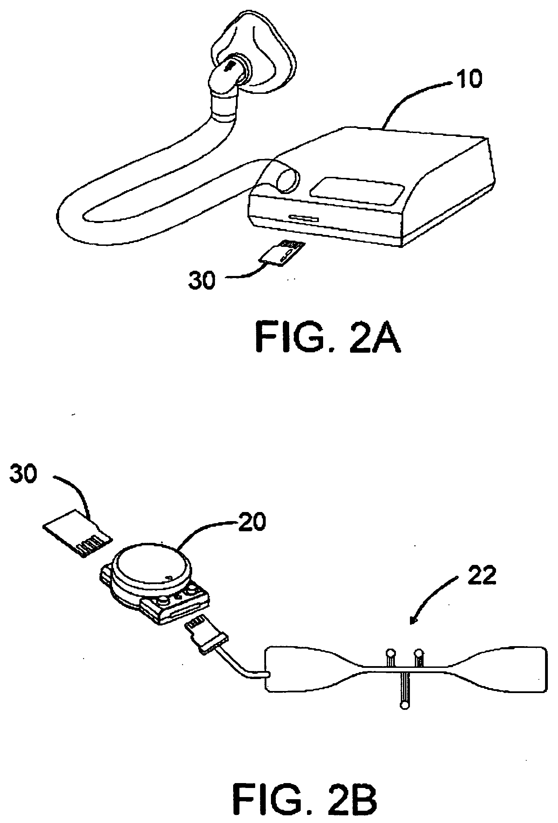

Please refer to Fig. 1 which is a circuit block showing the PAP device and the sensing device according to the present invention, and Figs. 2A-2B which show the PAP device and the sensing device in a preferred embodiment of the present invention. As shown, the PAP device 10 of the present invention includes a processor 11, a flow generator 12, a digital communication module 13 and a power source 14, wherein the processor 11 controls the PAP device, the flow generator 12 supplies a breathable pressured air to the patient according to an direction from the processor 11, the digital communication module 13 receives or sends out digital signals, and the power source 14 provides electricity for operation. Here, if the PAP device is used at home, the wall outlet is usually employed as the power source, and if the PAP device is used as traveling, the battery can be used as the power source, but there is no limitation.

Of course, for supplying breathable pressured air to the patient, the PAP device 10 also should connect to the patient interface, namely, the mask, the tubing and other related accessories, however, since the patient interface is well known in the art, it is omitted in the following descriptions.

Furthermore, the external sensing device 20 includes a processor 21, at least a sensor 22, a digital communication module 23, and a power source 24, wherein the processor 21 controls the sensing device 20, the sensor 22 acquires physiological information related to the patient according to directions from the processor 21, such as, from the body surface of the patient, or in the mask or the tubing, the digital communication module 23 executes an external digital communication, such as, with the digital communication module 13 of the PAP device 10, and the power source 24 provides the sensing device 20 the operation power. Here, the power source 24 can be a rechargeable battery, and correspondingly, the PAP device can have a battery holder for charging the battery. Alternatively, the sensing device 20 can connect to the PAP device through a connecting wire to acquire the power. Therefore, there is no limitation.

And, the digital communication modules 13,23 of the PAP device 10 and the sensing device 20 can be implemented to perform wired and/or wireless communication with each other or with an external device, such as, a computer or a handheld device, without limitation.

Besides, in addition to be the basic PAP device, which delivers constant pressure, the PAP device 10 also can be originally provided with the function of pressure adjustment, such as, a BiPAP device or an APAP device, and in this case, the PAP device can be implemented to provide the information for modifying the pressure adjustment, for example, the pressure value, the leakage or the respiratory cycle. That is, the PAP device according to the present invention can be all kinds of PAP devices, without limitation.

Followings describe the operation of the PAP device 10 and the external sensing device 20.

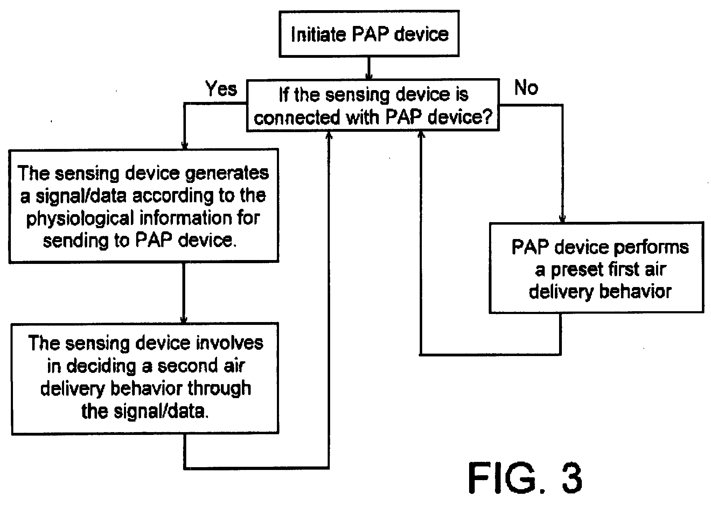

As shown in Fig. 3, which is a flow chart showing the operation of the extendable air delivery system of the present invention. First, after the PAP device 10 is turned on, the processor 11 will check if there is any device communicated with the digital communication module 13, for example, through USB connection or Bluetooth.
1. If there is no external connection found:
   The PAP device 10 enters a preset independent operation mode, that is, even without any external connection, the PAP device 10 still can operate accordingly. In the independent operation mode, the flow generator 12 performs a first air delivery behavior preloaded in the processor 11, so as to provide a breathable pressured air to the patient. Here, if the PAP device 10 is a CPAP device, the air delivery behavior will be delivering a breathable air having a fixed pressure, or if the PAP device 10 is a BiPAP device, the air delivery behavior will be delivering a breathable air varying between two pressures. Therefore, the original functions of the PAP device 10 will not be influenced.
2. If there finds a connection from the external sensing device 20:
   The PAP device 10 enters a common operation mode. In this mode, the PAP device 10 and the external sensing device 20 will commonly decide a second air delivery behavior of the PAP 10. During operation, the PAP device 10 will continuously check the connection from the sensing device 20, so as to accordingly change the operation mode.

As the connection of the external device triggers the common operation mode, the PAP device 10 and the external sensing device 20 will perform a negotiation process, e.g., authentication and handshaking, so as to make sure the capability of each other. After the negotiation process, it can decide that which kind of cooperation between the two will be, for example, which one takes control, or which kind of physiological signals will be provided by whom if both possess the capability of physiological signal acquisition.

After the cooperation is decided, the common operation starts.

During the common operation mode, the sensing device 20 will generate a signal/data according to the acquired physiological information, and the signal/data is transmitted to the PAP device 10 through the digital communication modules 13,23. Then, the received signal/data will involve in the decision for the second air delivery behavior, for example, the processor 11 of the PAP device 10 can make the decision by referring to the signal/data, or the sensing device 20 can control the air delivery behavior of the PAP device 10 via the signal/data.

Besides, the cooperation of the PAP device 10 and the sensing device 20 may also adopt the master-slave model. For example, when the PAP device and the sensing device 20 communicate with each other, the air pressure delivery can be controlled by the sensing device 20, by the PAP device 10, or by both, such as, at the same time, in turn or based on a given rule. There is no limitation.

In addition to the master-slave model described above, another situation can be that the PAP device 10 acquires physiological information, which is overlapped with those acquired by the sensing device 20. Then, in this case, the sensing device 20 can stop the acquisition of the overlapped information by the PAP device, or the overlapped information can be replaced by the information provided by the sensing device 20, or, particularly, it also can be that two information is compared to decide the more suitable one, for example, to compare the therapeutic effect for the patient. So, there is no limitation.

Other than providing the signal/data to the PAP device by the sensing device, through the digital communication, the PAP device also can provide an air delivery-related information to the sensing device to be the basis of the signal/data or the reference for controlling.

Therefore, through the independent and the common operation modes, the PAP device of any kind can regulate the air delivery behavior thereof by cooperating with the external sensing device, so as to match the patient's requirement.

For example, if the PAP device is originally a CPAP device which delivers constant pressure, by cooperating with different sensing devices, the CPAP device can be provided with additional capabilities for air delivery, so as to function as a BiPAP, an APAP, or a VPAP, or to obtain the information for executing/adjusting the ramp process. Identically, the BiPAP device, APAP device, or VPAP device also can obtain extra physiological information of the patient from the sensing device for further adjusting the air delivery. Thereby, the regulation of the air delivery behavior can be more accurate.

Then, because the sensing device 20 is separated from the PAP device 10, there is no limitation to the installation position of the sensing device 20. The principle for positioning the sensing device 20 is to provide an accurate physiological information which can directly reflect the variation of patient's physiological condition, so the second air delivery behavior which is commonly decided through the sensing device can approach to the patient's real breathing pattern even more, thereby improving the using comfortability and reducing the occurrence of arousal.

Consequently, the operation of the extendable air delivery system according to the present invention is dynamic. The PAP device 10 not only will keep checking the connection with the sensing device 20 to decide the operation mode, but also will interact with the sensing device 20 to dynamically adjust the air delivery for obtaining a better therapeutic effect.

Then, how the sensing device 20 decides the air delivery of the PAP device 10 is described below.

Generally, the physiological information which can serve as the basis for the sensing device 20 to generate the signal/data includes, but not limited, the respiratory cycle, the occurrence of sleep apnea/hypopnea, the sleep stage and the body position. These physiological information can help to understand the breathing pattern and the physiological condition of the patient.

Usually, the information for deciding the respiratory cycle includes, but not limited, respiratory effort, air flow, flow/pressure in the mask, flow/pressure in the tubing, and EMG on torso; the information for deciding the occurrence of sleep apnea/hypopnea includes, but not limited, respiratory effort, air flow, snore, oxygen saturation, and heart rate; the information for deciding the sleep stage includes, but not limited, EEG signals, EOG signals, EMG signals, and HRV; and the information for deciding the body position includes, but not limited, body position, limb movement, and EMG signals.

In addition, an important aspect of air pressure delivery during the treatment is the ramp process, which is a smooth pressure-rising process before the delivered pressure comes to the preset level, namely, the therapeutic pressure. The ramp process can provide the patient a more comfortable period before falling asleep with a lower pressure level. Here, the information for deciding the ramp process includes, but not limited, EEG, EOG, EMG and heart rate.

Therefore, in the present invention, the external sensing device 20 plays the role of providing the information for deciding the behavior of air delivery. And, as the sensing devices 20 employs different sensors, different signals/data can be generated to conform to different requirements.

Particularly, in the present invention, the flow/pressure sensor combined with the mask or the tubing can directly detect the flow/pressure variation in the mask/tubing, so that the calculation for obtaining the leakage as using the conventional flow/pressure sensor which is mounted in the PAP device can be omitted.

Besides, advantageously, since some physiological signals, such as, airflow, EEG, EOG, EMG, forehead/ear SPO2, and snore, are acquired from positions around the patient's head, which is close to the mask, it is more convenient for the patient to combine the sensing device/the sensor on the mask, for example, to combine the air flow sensor with the mask itself or combine the EEG electrodes with the headgear for fixing the mask.

Of course, the descriptions above are only for illustration, and not for limitation, and other factors and physiological information that can be used to adjust the delivery pressure all should belong to the present invention.

The following Table 1 lists the physiological information, the employed sensor for acquiring thereof and the sleep information corresponding thereto. However, as mentioned, the table is for illustration, not for limitation.

**Table 1**

| **PHYSIOLOGICAL INFORMATION** | **SENSOR** | **RELATED SLEEP INFORMATION** |
|---|---|---|
| ECG | ECG electrodes | Sleep apnea/hypopnea |
| EEG | EEG electrodes | Sleep stage |
| EMG | | |
| EMG on chin | | Sleep stage |
| EMG on torso | EMG electrodes | Respiratory effort |
| EMG on other positions | | Body position/movement |
| EOG | EOG electrodes | Sleep stage |
| Air flow | Thermal sensor (e.g., thermistor, thermal coupler) Pressure transducer | Respiratory cycle Sleep apnea/hypopnea |
| Respiratory effort | Piezoelectric pressure sensor, RIP sensor, EMG electrodes on torso | Respiratory cycle Sleep apnea/hypopnea |
| Snore | Microphone, piezoelectric pressure sensor, PVDF | Sleep apnea/hypopnea related |
| Oxygen saturation | PPG sensor (photoplethysmography) | Sleep apnea/hypopnea |
| Body position | Accelerator | Body position |
| Limb movement | Accelerator, EMG electrodes | Body/limb movement |
| Flow/pressure in mask | Pressure/flow sensor | Respiratory cycle, pressure in the mask |
| Flow/pressure in tubing | Pressure/flow sensor | Respiratory cycle, Pressure in the tubing |
| Heart rate | ECG electrodes, Oximeter | Sleep apnea/hypopnea, Sleep stage (e.g., HRV analysis) |

Therefore, when the external sensing device 20 connects to different kinds of sensors 22, the PAP device 10 will receive different kinds of signals/data, and based on the communication therebetween and according to the received signal/data, the PAP device 10 can perform different patterns of air delivery, so as to provide more suitable air delivery pressure.

That is, through the external sensing device 20 of the present invention, the flexibility of the PAP device 10 can be increased, and different from the conventional architecture, the present invention significantly improve the adaptation of the PAP device to all kinds of patients.

In addition, the type and the quantity of the sensor 22 connected to the sensing device 20 are also not restricted. The sensor 22 can be varied according to different patients' demands. For example, if it wishes to decide the air delivery pressure based on the occurrence of sleep apnea/hypopnea, the sensor can be selected to be, but not limited, an air flow sensor with a snore sensor. Then, if the pressure delivery based on different sleep stages is required, the sensing device can further connect with EEG/EOG/EMG electrodes. That is, when the sensing device involves in deciding the air delivery, single signals/data can be related to single type or multiple types of physiological information.

Of course, the number of the sensing device 20 is also not restricted. For example, the multiple sensors as described above can be respectively connected to several sensing devices, and each sensing device generates and sends its own signal/data to the PAP device, so as to achieve a situation that multiple sensing devices cooperate with the PAP device. Here, in addition to each sensing device communicates with the PAP device independently, it also can be one of the sensing devices serves as the control master for organizing the communications therebetween. Therefore, there is not limitation.

Then, the regulation of the air delivery under the cooperation of the PAP device and the sensing device 20 is described as below.

First at all, it should be noticed normally the PAP device (CPAP, BiPAP, APAP, VPAP etc.) operates within a pressure range according to the settings, but with the confirmation of the doctor/technician, it is also possible that the sensing device can regulate the pressure limits as needed.

In the common operation mode, the cooperation therebetween can be that the sensing device provides the physiological information and the PAP device decides the air delivery behavior according thereto.

Alternatively, the cooperation also can be the sensing device directly controls the delivery pressure by sending the signal/data as a command to the PAP device.

One possible way is the signal/data causes the PAP device to increase/decrease the delivered pressure. For example, if the sensing device found that the patient had a high RDI/AHI value, which means the delivered pressure is too low, the sensing device can control the flow generator to increase the pressure via the signal/data until the recalculated RDI/AHI value falls in the preset range. Here, as long as the sensing device keeps sending the signal/data, the air delivery behavior will be modified continuously. Accordingly, the record of the air delivery behaviors over the whole process provides a pressure-time profile.

Another possible way is the signal/data provides an absolute target pressure or a value of pressure increment/decrement for the PAP. For example, the signal/data can directly indicate the PAP device to achieve a delivery pressure of 7 cmH₂O, or to increase/decrease a delivery pressure of 1 cmH₂O. Another possible way is to represent the pressure increment/decrement in percentage. For example, the PAP device can be directed to decrease 10% the present pressure, or to decrease to 30% the upper pressure limit. Still another possible way is to provide an algorithm related to a preset pressure, so that the PAP device can obtain the required pressure via calculation.

Moreover, in a preferred embodiment, when the PAP device is a BiPAP, the signal/data can be used to evaluate the suitability of the pressure values needed during the inspiration and the expiration periods (i.e. IPAP and EPAP). Further, if the signal/data is generated based on physiological information other than the respiratory cycle, such as, the sleep stage, then the signal/data also can shift the IPAP and EPAP to match different sleep stages. Therefore, based on the regulation mechanism of the present invention, the BiPAP device can achieve a better therapeutic effect.

It should be noticed that the pressure regulation above is only for illustration, not for limitation, and any kind of mechanism achieved by the cooperation between the PAP device and the sensing device to regulate the delivery pressure may fall in the scope of the present invention.

Furthermore, the PAP device 10 and/or the sensing device 20 also can equip with a memory 30 for storing the recorded operation data of the whole system. Here, the memory can be a built-in or removable memory.

Through the memory, the doctor/technician can have the opportunity to understand the treatment processes and the patient's physiological condition.

It is known that the respiration pattern will be different according to physiological condition, such as, weight loss or weight gain, so it will be better to check if the settings of the PAP device are still suitable for the patient's physiological condition frequently. Therefore, if the therapeutic effect departs from the expectation, the patient may need to ask for doctor's opinion. And, through the memory, the doctor can easily access the records. Besides, owing to the sensor(s) of the sensing device, not only the behavior of the delivery pressure, other useful information, such as, the sleep stage and/or the body position, during the treatment also can be recorded, so that the doctor can have a more comprehensive understanding about the patient. Further, if the memory is implemented to be removable, the patient can visit the doctor by only carrying the memory. Then, if the doctor wants to modify the settings of the PAP device, the changes can be directly stored in the memory for updating.

In another embodiment, the PAP device/the sensing device can be implemented to show the treatment result, such as, RDI/AHI value (e.g., through the algorithm preloaded in the sensing device) after each operation, so that the patient can easily check the therapeutic effect and review the settings of the whole system. For example, the sensing device (sensors) is selected improperly or the patient's physiological condition has changed, e.g., weight loss or gain, to influence the therapeutic effect, so it might consider to modify the settings or to add another sensor (sensing device) or replace the original sensor (sensing device).

In another aspect of the present invention, the extendable and adjustable architecture also can simplify the titration process.

The traditional titration process for the PAP device is the patient uses the PAP device in the sleep lab with installing multiple sensors at the same time, so that the doctor/technician can adjust the delivered pressure to a proper range based on the information acquired by the sensors. For example, by obtaining the RDI (Respiratory Disturbance Index)/AHI (Apnea/Hypopnea Index) value, the technician/doctor can evaluate the therapeutic result. Moreover, in addition to treating the obstruction, another important purpose of titration is to find the minimum effective delivery pressure, so as to reduce the occurrence of arousal and also provide the patient the most comfortable using experience. And, the sensors usually used in the titration process are those listed in Table 1.

However, the traditional titration process has some inconveniences. Since the PAP device can not directly communicate with the sensors, the acquired physiological information must be analyzed by the doctor/technician to obtain the desired pressure value for inputting into the PAP device.

Oppositely, according to the present invention, because the sensing device can have a digital communication with the PAP device for directly providing the acquired physiological information to the PAP device and further because the sensing device is exchangeable and extendable, the titration process becomes more convenient for the doctor/technician.

Furthermore, if the wireless communication capability, as described above, is used in the titration process, the doctor/technician can experience the convenience even more.

Through wireless communication, during the titration process, the PAP device/the sensing device can transmit the information about respiration, delivery pressure and others to an external device, so that the doctor/technician can, in real time, monitor the data, such as, the RDI/AHI value, and modify the settings of the PAP device.

More advantageously, based on the extensibility and flexibility of the present invention, when the titration process is performed in the sleep lab, for providing more comprehensive information to define an accurate range of delivery pressure, more sensing devices can be employed. And, on the other hand, as being used at home, the PAP device can cooperate with fewer sensing device(s) since the proper pressure range has been found during the titration process.

Also, according to the present invention, since the cooperation between the PAP device and the sensing device can automatically decide the air delivery pattern, a home titration becomes possible. And, when professional opinions are needed on determining the pressure settings, the memory can provide the necessary information.

Furthermore, via the memory, the system can collect and analyze the long-term operation data to obtain an even more suitable air delivery since the more comprehensive the data, the more accurate the obtained air delivery pattern.

Here, the removable memory can be a standard memory card or IC card, such as, SD, CF, MS, MMC, xD card, or smart card, and with a corresponding interface, e.g., card reader, the data stored in the removable memory can be accessed by a computer or other devices. Further, when the computer or device for accessing the removable memory connects to the network, the data stored in the memory can be uploaded, and oppositely, the patient can download data via the network to the memory. Similarly, as the memory is a built-in memory, it can be implemented as the PAP device/the sensing device directly connects to the computer/device with network connection to perform the data access, upload, and download.

In addition to connecting to the network via the computer/device, according to another preferred embodiment of the present invention, the air delivery system can also equip with a communication interface for network connection. For example, through a built-in network interface, or a serial port connected to a modem, the PAP device/the sensing device can connect to the network to perform a remote communication.

By the network connection, the patient can upload the data (such as, the physiological information and the pressure related information) at any time to, for example, a remote doctor/technician, or a website being accessed by the doctor/technician, or a website which provides algorithms for analysis, so that the doctor/technician and/or the website can provide related opinions/diagnosis to help the patient to modify the settings of his/her own air delivery system (the PAP device and the sensing device).

Similarly, in addition to obtaining the opinions/diagnosis from the doctor/technician and/or the website, downloading the settings and manipulating the operation of the PAP device/sensing device are also possible. For example, if at the beginning, the sensing device adopts airflow signals to regulate the air delivery and the treatment effect departs from the expectation after a period of time, then the patient can choose another kind of physiological signals, such as, ECG signals, for being the reference to the adjustment. And, in this case, the patient can download the related ECG program via the network to replace the original airflow program or to enable the sensing device to receive multiple kinds of signals (if applicable). Therefore, the air delivery behavior can be easily changed only through replacing or adding the sensor without replacing/adding the sensing device. The cost can be effectively reduced without losing the system flexibility

Followings are exemplary illustrations for describing the applications of the present invention, and as known, not for limitation.

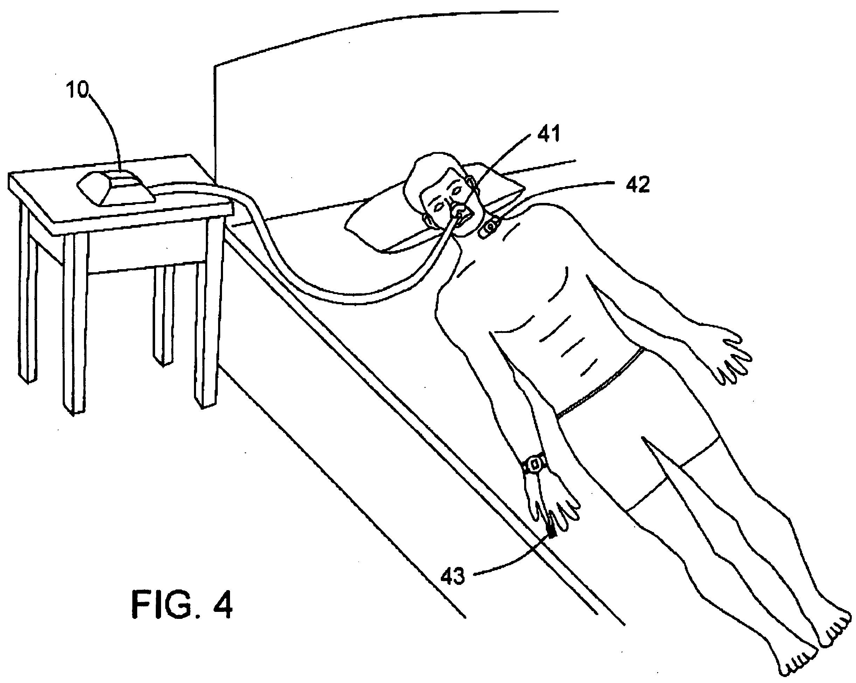

As shown in Fig. 4, supposed that the PAP device 10 is a CPAP device which delivers a breathable air of constant pressure cooperating with an airflow sensing device 41 and/or a snore sensing device 42 and a SPO2 sensing device 43, the extendable air delivery system according to the present invention can function as an APAP (auto PAP) device. Since the APAP device takes the occurrence of apnea/hypopnea as the basis for regulating the delivery pressure, when the external sensing devices provide the information about apnea/hypopnea, this system acts as an APAP device. Here, the airflow signals can help to determine the occurrence of apnea/hypopnea, the snore signals can be used to check the obstruction of the upper airway, and the SPO2 signals can indicate the desaturation of blood oxygen which represents the oxygen insufficiency caused by the apnea/hypopnea.

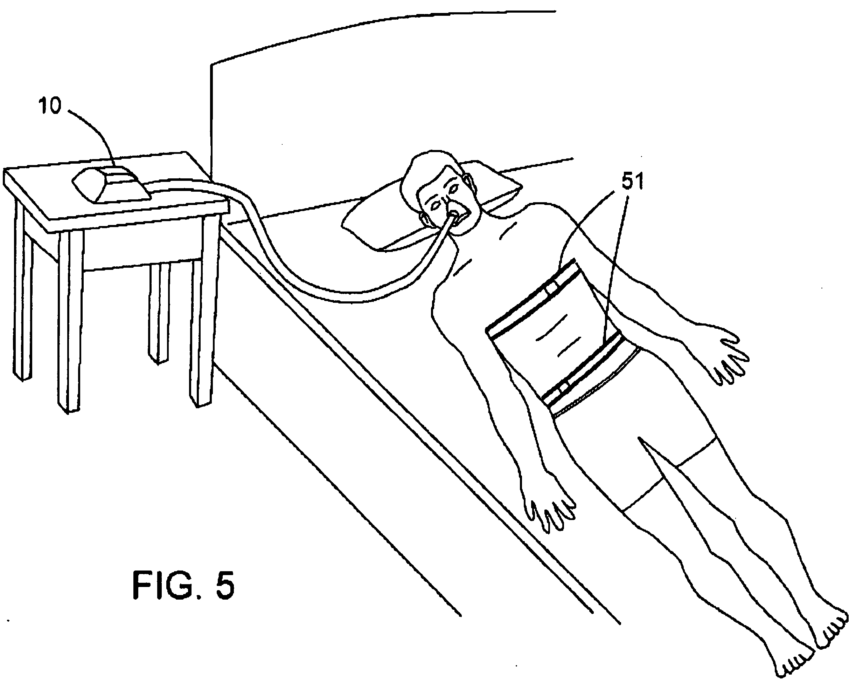

In another example, as shown in Fig. 5, the PAP device 10 is a CPAP for delivering a breathable air with constant pressure, and the sensing device 51 is implemented to acquire physiological signals of respiratory effort, such as, by using RIP (Respiratory Inductive Plethysmograph) belts (as shown, the thoracic and abdominal belts), so that the extendable air delivery system according to the present invention can function as a BiPAP (Bi-Level PAP) device. As known, the BiPAP device delivers different pressures in accordance with the patient's inspiration and expiration during the treatment, so that when the thoracic and abdominal belts provide the information about respiratory effort for defining the inspiration and the expiration, the PAP device can function as a BiPAP device.

According to the two examples above, it can be seen, through cooperating with the external sensing device, even the CPAP device can be improved to function ase the APAP device, or the BiPAP device, or other kinds of air delivery method. More particularly, because according to the present invention, the type and the quantity of the sensor can be selected to match each different demand, the degree of customization can be maximized.

Moreover, the separation of the PAP device and the sensing device also provide the opportunity to attain the goal of home monitoring.

Since there is no restriction on the type and number of the sensor and the sensing device, when the sensing device(s) is used independently, it becomes a general home use physiological signal acquisition device.

For example, if the sensing device(s) is only connected with few sensors, such as, an airflow sensor only, or an airflow sensor plus a snore sensor, or an airflow sensor plus a SPO2 sensor, then the sensing device(s) with sensor(s) can be regarded as a sleep screener. Alternatively, when the collection of sensors of all sensing devices is equivalent to those of a PSG (polysomnograph) device, a residential PSG system can be achieved. Then, through the analysis capability of the sensing device and/or the analysis/calculation capability of an external device (e.g., a dedicated processing device, or a computer) connected to the sensing device (or for accessing the removable memory of the sensing device), and/or the analysis/calculation capability obtained from a remote device via the network (by way of the sensing device or the external processing device), the analysis result, evaluation and/or diagnosis can be obtained. Naturally, the sensing device and/or the PAP device can include a display unit to show the result.

Further, since the present invention provides the possibility to increase/decrease the type and number of the sensing device/the sensor, the home monitoring is also extendable. So, the cost can be effectively saved owing to the multifunctional property of the present air delivery system.

From another point of view, in case the patient already owns the physiological acquisition device (a sleep screen or PSG) at home, by cooperating with a PAP device which can receive the signals therefrom and perform the delivery regulation according thereto, the extendable system also can be established.

In the aforesaid, the extendable air delivery system according to the present invention provides two communicable parts, a PAP device and at least a sensing device, to offer patients a possibility to decide the function of their own PAP device, in which the function of the PAP device is decided by the type and the number of the sensing device (the sensor), so that the patients are no longer restricted by the unchangeable hardware structure and air delivery behavior of the given PAP device, for example, when the patient has a stuffy nose, the snore sensor obviously becomes improper and should be replaced. Therefore, the selectability/adjustability of the sensing device (sensor) provides the opportunity to optimize the air delivery behavior.

Moreover, through the recorded operation data stored in the memory (such as, system settings, pressure delivery profile, and respiration profile), the discussion with the doctor and the modification from the doctor both become easier, especially through the removable memory. And, owing to the digital communication between the PAP device and the sensing device and with the external device, the present system provides a simplified and convenient titration process. Plus, the system of the present invention also can be designed to record the operation data for a long period of time before deciding the delivery behavior, therefore, a better delivery behavior to fit the patient's demand can be obtained by extensive data analysis. More advantageously, the patient can perform the titration process at home (via the network) rather than spending a whole night in the lab.

Furthermore, the extendable air delivery system of the present invention also can connect to the network, so that the patient can upload the operation data (to the website/the doctor) for checking the therapeutic effect, and also, the PAP device/the sensing device can be updated by downloading new operation program, so as to match different requirements.

Consequently, through offering the possibility to adjust the type and the number of the sensing device (sensor), the extendable air delivery system of the present invention can provide a more customized air delivery behavior for every patient, thereby improving the comfortability of usage and also reducing the occurrence of arousal. Besides, as idling the PAP device, the sensing device alone becomes a home use physiological signal acquisition device, which can provide the patient useful physiological information to make the present invention a multifunctional system.

The above examples and disclosure are intended to be illustrative and not exhaustive. These examples and description will suggest many variations and alternatives to one of ordinary skill in this art. All these alternatives and variations are intended to be included within the scope of the attached claims. Those familiar with the art may recognize other equivalents to the specific embodiments described herein which equivalents are also intended to be encompassed by the claims attached hereto.

## Claims

1. An extendable air delivery system, including a PAP device (10) and a sensing device (20), wherein:
the PAP device (10) comprises:
a flow generator (12), for providing a breathable air to a patient;
a processor (11), for providing a control signal to the flow generator (12); and
a digital communication module (13), controlled by the processor (11); and
the sensing device (20) comprises:
at least a sensor (22), for acquiring a physiological information from the patient;
a processor (21), for obtaining the physiological information from the sensor (22) and generating a signal/data according thereto; and
a digital communication module (23), controlled by the processor (21),
the PAP device is configured to be one selected from a CPAP device, a BiPAP device, an APAP device and a VPAP device, and the PAP device (10) and the sensing device (20) are configured to be connected to or separated from each other ; and wherein
when the sensing device (20) is not connected with the PAP device (10), the PAP device (10) enters a preset independent operation mode and performs a preloaded first air delivery behavior of CPAP device, or BiPAP device, or APAP device, or VPAP device; and
when the sensing device (20) is connected with the PAP device (10), the PAP device (10) enters a common operation mode, and during the common operation mode, the PAP device (10) and the sensing device (20) communicate via the corresponding digital communication modules thereof, for sending the signal/data from the sensing device (20) to the PAP device (10), thereby deciding a second air delivery behavior of the flow generator (12), wherein the second air delivery behavior is an air delivery behavior of BiPAP device, or APAP device, or VPAP device; and
the PAP device is configured to check the connection with the sensing device to decide the operation mode.

2. The system as claimed in claim 1, **characterized in that** the processor of the PAP device (10) provides an air delivery-related information to the sensing device (20) to involve in the generation of the signal/data.

3. The system as claimed in claim 1, **characterized in that** the digital communication modules (13, 23) are wired or wireless.

4. The system as claimed in claim 1, **characterized in that** the processor of the sensing device (20) is implemented to analyze the physiological information for providing an information about the patient's physiological condition.

5. The system as claimed in claim 1, **characterized in that** the PAP device (10) and/or the sensing device (20) is able to communicate with an external device for further processing, setting, and/or analysis.

6. The system as claimed in claim 5, **characterized in that** the external device is implemented to connect to a network.

7. The system as claimed in claim 1, **characterized in that** the PAP device (10) and/or the sensing device (20) is implemented to connect to a network.

8. The system as claimed in claim 1, **characterized in that** a plurality of sensing devices (20) is provided and configured to provide single or multiple signal/data related to single kind or multiple kinds of physiological information.

## Patentansprüche

1. Erweiterbares Luft-Zufuhr System, das eine PAP-Vorrichtung (10) und eine Sensor-Vorrichtung (20) umfasst, wobei:
die PAP-Vorrichtung (10) Folgendes umfasst:
einen Fluss-Erzeuger (12), der dem Patienten atembare Luft zur Verfügung stellt;
einen Prozessor (11), der dem Fluss-Erzeuger (12) ein Steuersignal zur Verfügung stellt; und
ein digitales Kommunikationsmodul (13), das durch den Prozessor (11) gesteuert wird; und wobei
die Sensor-Vorrichtung (20) Folgendes umfasst:
mindestens einen Sensor (22) zum Erlangen von physiologischen Informationen des Patienten;
einen Prozessor (21) zum Erlangen der physiologischen Information von dem Sensor (22) und zum Erzeugen eines dazugehörigen Signals/Werts ; und
ein digitales Kommunikationsmodul (23), das durch den Prozessor (21) gesteuert wird,
**gekennzeichnet dadurch, dass**
die PAP-Vorrichtung beschaffen ist als eine CPAP-Vorrichtung, eine BiPAP-Vorrichtung, eine APAP-Vorrichtung oder eine VPAP-Vorrichtung ausgewählt zuwerden, wobei die PAP-Vorrichtung (10) und die Sensor-Vorrichtung (20) konfiguriert sind, um miteinander verbunden oder voneinander getrennt zu sein; und wobei,
wenn die Sensor-Vorrichtung (20) nicht mit der PAP-Vorrichtung (10) verbunden ist, die PAP-Vorrichtung (10) in einen aktuellen unabhängigen Operationsmodus eintritt und ein vorgeladenes erstes Luft-Zufuhr-Verhalten der CPAP-Vorrichtung, der BiPAP-Vorrichtung, der APAP-Vorrichtung oder der VPAP-Vorrichtung durchführt; und
wenn die Sensor-Vorrichtung (20) mit der PAP-Vorrichtung (10) verbunden ist, die PAP-Vorrichtung (10) in einen gemeinsamen Operationsmodus eintritt und dass, während des gemeinsamen Operationsmodus, die PAP-Vorrichtung(10) und die Sensor-Vorrichtung (20) über die dazugehörige digitale Kommunikationsmodule zu kommunizieren, um das Signal/den Wert von der Sensor-Vorrichtung (20) zu der PAP-Vorrichtung (10) zu senden und dabei über ein zweites Luft-Zufuhr-Verhalten des Fluss-Erzeugers (12) zu entscheiden, wobei das zweite Luft-Zufuhr-Verhalten ein Luft-Zufuhr-Verhalten einer BiPAP-Vorrichtung, einer APAP-Vorrichtung oder einer VPAP-Vorrichtung ist; und wobei
die PAP-Vorrichtung konfiguriert ist zur Überprüfung der die Verbindung mit der Sensor-Vorrichtung, um über den Operationsmodus zu entscheiden.

2. System nach Anspruch 1, **gekennzeichnet dadurch, dass** der Prozessor der PAP-Vorrichtung (10) Informationen in Bezug auf die Luft-Zufuhr an das Sensor-Vorrichtung (20) zur Verfügung stellen, um diese Informationen in die Erzeugung des Signals/Wertes mit einzubeziehen.

3. System nach Anspruch 1, **gekennzeichnet dadurch, dass** die digitalen Kommunikationsmodule(13, 23) durch Kabel oder kabellos verbunden sein können.

4. System nach Anspruch 1, **gekennzeichnet dadurch, dass** der Prozessor des Sensor-Vorrichtungs (20) eingebaut ist, um physiologischen Informationen zu analysieren, und um Informationen über den physiologischen Zustand des Patienten zur Verfügung zu stellen.

5. System nach Anspruch 1, **gekennzeichnet dadurch, dass** die PAP-Vorrichtung (10) und/oder das Sensor-Vorrichtung (20) in der Lage ist, mit einer externen Vorrichtung zu kommunizieren, um weiter zu verarbeiten, einzustellen und/oder zu analysieren.

6. Das System nach Anspruch 5, **gekennzeichnet dadurch, dass** die äußere Vorrichtung eingebaut ist, um sich mit einem Netzwerk zu verbinden.

7. Das System nach Anspruch 1, **gekennzeichnet dadurch, dass** die PAP-Vorrichtung (10) und/oder das Sensor-Vorrichtung (20) eingebaut ist, um sich mit einem Netzwerk zu verbinden.

8. Das System nach Anspruch 1, **gekennzeichnet dadurch, dass** eine Vielzahl von Sensor-Vorrichtungen (20) zur Verfügung gestellt werden und konfiguriert sind, um eine Einzahl oder eine Vielzahl von Signalen/Werten zur Verfügung zu stellen, die in Relation zu einer Art von physiologischen Informationen oder mehreren Arten von physiologischen Informationen stehen.

## Revendications

1. Un système de distribution d'air extensible, comprenant un dispositif PAP (10) et un dispositif de détection (20), dans lequel
le dispositif PAP (10) comporte :
un générateur de flux (12), pour fournir de l'air respirable à un patient ;
un processeur (11), pour fournir un signal de commande au générateur de flux (12) ; et
un module de communication numérique (13), commandé par le processeur (11) ; et
le dispositif de détection (20) comporte :
au moins un capteur (22) ; pour l'acquisition d'une information physiologique du patient ;
un processeur (21), pour l'obtention de l'information physiologique du capteur (22) et pour en générer un signal/données ; et
un module de communication numérique (23), commandé par le processeur (21),
le dispositif PAP est configuré pour être sélectionné à partir d'un dispositif CPAP, un dispositif BiPAP, un dispositif APAP et un dispositif VPAP, et le dispositif PAP (10) et le dispositif de détection (20) sont configurés pour être connectés à ou séparés les uns des autres ; et dans lequel
lorsque le dispositif de détection (20) n'est pas connecté au dispositif PAP (10), le dispositif PAP (10) entre dans un mode opératoire indépendant de pré-réglage et effectue une première distribution d'air préchargée du dispositif CPAP, ou du dispositif BiPAP, ou du dispositif APAP, ou du dispositif VPAP ; et
lorsque le dispositif de détection (20) est connecté au dispositif PAP (10), le dispositif PAP (10) entre dans un mode opératoire commun, et durant le mode opératoire commun, le dispositif PAP (10) et le dispositif de détection (20) communiquent via les modules de communication numériques correspondants, pour la transmission du signal/données depuis le dispositif de détection (20) vers le dispositif PAP (10), permettant ainsi la décision d'une seconde distribution d'air du générateur de flux (12), dans lequel la seconde distribution d'air est une distribution d'air du dispositif PiPAP, ou du dispositif APAP, ou du dispositif VPAP ; et
le dispositif PAP est configuré pour tester la connexion avec le dispositif de détection pour la décision du mode opératoire.

2. Le système tel que revendiqué dans la revendication 1, **caractérisé en ce que** le processeur du dispositif PAP (10) fournit une information relative à la distribution d'air au dispositif de détection (20) pour être prise en compte dans la génération du signal/données.

3. Le système tel que revendiqué dans la revendication 1, **caractérisé en ce que** les modules de communication numériques (13, 23) sont filiaires ou sans fil.

4. Le système tel que revendiqué dans la revendication 1, **caractérisé en ce que** le processeur du dispositif de détection (20) est implémenté pour l'analyse de l'information physiologique pour fournir une information concernant la condition physiologique du patient.

5. Le système tel que revendiqué dans la revendication 1, **caractérisé en ce que** le dispositif PAP (10) et/ou le dispositif de détection (20) est capable de communiquer avec un dispositif externe pour un traitement supplémentaire, un réglage et/ou une analyse.

6. Le système tel que revendiqué dans la revendication 5, **caractérisé en ce que en ce que** le dispositif externe est implémenté pour une connexion à un réseau.

7. Le système tel que revendiqué dans la revendication 1, **caractérisé en ce que** le dispositif PAP (10) et/ou le dispositif de détection (20) est implémenté pour la connexion à un réseau.

8. Le système tel que revendiqué dans la revendication 1, **caractérisé en ce qu'**une pluralité de dispositifs de détection (20) est fournie et configurée pour fournir un seul ou une pluralité de signaux/données relative à un seul ou à une pluralité de types d'informations physiologiques.
